(19) Europäisches Patentamt
European Patent Office
Office européen des brevets

(11) **EP 3 228 343 A1**

(12) # EUROPEAN PATENT APPLICATION

(43) Date of publication:
**11.10.2017 Bulletin 2017/41**

(51) Int Cl.:
**A61M 5/168** (2006.01)

(21) Application number: **16305400.0**

(22) Date of filing: **06.04.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fresenius Vial SAS**
**38590 Brézins (FR)**

(72) Inventor: **WOLFF, Rémy**
**38210 Morette (FR)**

(74) Representative: **Kusche, Robert**
**Fresenius Kabi Deutschland GmbH**
**Patent Department Medical Devices Division**
**Else-Kröner-Straße 1**
**61352 Bad Homburg (DE)**

(54) **METHOD FOR OPERATING AN INFUSION DEVICE**

(57) A method for operating an infusion device (1) for administering a medical fluid to a patient (P) comprises: pumping, using a pumping mechanism (11), a fluid in a downstream direction through a delivery line (3); measuring, using a sensor device (14), a measurement value indicative of a pressure in the delivery line (3) in order to be able detect, from a rise of the pressure beyond a predefined pressure threshold, the presence of an occlusion in the delivery line (3); and, in case of an occlusion, reversing the pumping mechanism (11) to pump fluid in an upstream direction opposite the downstream direction for reducing the pressure in the delivery line (3). Herein, for stopping the reverse pumping of the fluid in the upstream direction at least two criteria are applied and the reverse pumping is stopped if at least one of the at least two criteria is fulfilled, wherein as a first criteria it is determined whether the pressure in the delivery line (3) has fallen below a predetermined residual pressure threshold, and as a second criteria it is determined whether the volume pumped in the upstream direction during the reverse pumping action of the pumping mechanism (11) has reached a predetermined volume computed based on the predefined pressure threshold used for the occlusion detection. In this way a method for operating an infusion device for administering a medical fluid to a patient is provided which allows for a precise and reliable stopping of a reverse pumping in order to obtain a desired reduction in pressure in the delivery line in case of an occlusion.

FIG 1

EP 3 228 343 A1

**Description**

The invention relates to a method for operating an infusion device for administering a medical fluid to a patient according to the preamble of claim 1 and to an infusion device for administering a medical fluid to a patient.

Within a method of this kind a fluid is pumped in a downstream direction through a delivery line using a pumping mechanism. During the pumping operation a measurement value indicative of a pressure in the delivery line is measured using a sensor device in order to be able detect, from a rise of the pressure beyond a predefined pressure threshold, the presence of an occlusion in the delivery line. In case of an occlusion, then, the pumping mechanism is reversed to pump fluid in an upstream direction opposite the downstream direction for reducing the pressure in the delivery line.

The method generally is applicable to operating infusion devices in the shape of syringe pumps or in the shape of volumetric (peristaltic) infusion pumps.

Within a syringe pump, a medical fluid (such as a medication or a nutritional fluid for example for the parenteral feeding of a patient) is contained in a cylindrical tube of a syringe. By continuously pushing a piston of the syringe into the cylindrical tube the medical fluid is delivered out of the cylindrical tube through a suitable delivery line towards a patient for infusing the medical fluid into the patient.

In a volumetric (peristaltic) infusion pump, in contrast, a pumping mechanism acts, in a peristaltic fashion, onto a delivery line, for example onto a pump segment of the delivery line comprising a flexible membrane, such that fluid is pumped through the delivery line towards a patient.

Within a syringe pump, a force sensor is for example placed on a driving element (a so called pusher device) of the pumping mechanism of the infusion device acting onto the piston of the syringe. By measuring the force exerted on the piston the pressure within the cylindrical tube of the syringe (which is connected to the delivery line) can be derived.

In a volumetric pump, in contrast, a pressure may be measured for example directly on the delivery line using a suitable force sensor, which is placed on the pump such that it is in contact with the delivery line.

Generally, the pressure within the delivery line during a normal infusion process can be assumed to be (almost) 0, due to the resistance for delivering the medical fluid towards the patient being small. However, if an occlusion is present in the delivery line downstream of the pumping device, the pressure in the delivery line will rise, which can be detected via the force sensor and which can be used to trigger an alarm if the pressure exceeds a certain preset threshold value.

If an occlusion occurs, the occlusion should be released by a user, for example a nurse, as fast as possible in order to continue infusion. The occlusion release hence may take place suddenly, which may lead, due to the pressure that has developed in the delivery line because of the occlusion, to a bolus being delivered to the patient. The bolus may have a significant volume and hence generally should be avoided.

Therefore, upon detection of the occlusion, it is desired to reduce the pressure in the delivery line prior to releasing the occlusion. For this, upon detection of an occlusion the pumping mechanism of the infusion device is reversed in its pumping action such that fluid is pumped in the upstream direction until a sufficient reduction in pressure is obtained.

One issue that arises in this regard is however that it must be determined when to stop the reverse pumping action of the pumping mechanism. If the reverse pumping is stopped too early, a significant pressure may remain in the delivery line, leading to a bolus of an unacceptable volume. If the reverse pumping however is stopped too late, a negative pressure may develop in the delivery line, leading to a backflow of fluid in the upstream direction through the delivery line, which also should be avoided.

It is an object of the instant invention to provide a method for operating an infusion device for administering a medical fluid to a patient and an infusion device for administering a medical fluid to a patient which allow for a precise and reliable stopping of a reverse pumping in order to obtain a desired reduction in pressure in the delivery line in case of an occlusion.

This object is achieved by means of a method comprising the features of claim 1.

Accordingly, for stopping the reverse pumping of the fluid in the upstream direction at least two criteria are applied and the reverse pumping is stopped if at least one of at least two criteria is fulfilled, wherein

- as a first criteria it is monitored whether the pressure in the delivery line has fallen below a predetermined residual pressure threshold, and
- as a second criteria it is monitored whether the volume pumped in the upstream direction during the reverse pumping action of the pumping mechanism has reached a predetermined volume computed based on the predefined pressure threshold used for the occlusion detection.

Hence, at least two criteria are applied to stop the reverse pumping for reducing the pressure in the delivery line in case of an occlusion. These (at least) two criteria are continuously examined, and if at least one of the at least two criteria is fulfilled, the reverse pumping is stopped.

By applying (at least) two criteria, a reliable stopping of the reverse pumping can be obtained, leading to a sufficient reduction in pressure in the delivery line. Herein, the reduction in pressure shall be such that a development of a negative pressure in the delivery line is avoided and a potential bolus is reduced to an acceptable (small) volume.

The first criteria may be denoted as a pressure target. Namely, within the first criteria it is observed whether the pressure in the delivery line has fallen below a predetermined residual pressure threshold. For this, from the measurement values taken by the sensor device the pressure in the delivery line is derived and is observed, and if it is found that the pressure has become smaller than a predetermined residual pressure threshold, the first criteria is fulfilled.

The residual pressure threshold herein, in one aspect, is chosen positive. The residual pressure threshold hence corresponds to a positive pressure in the delivery line which may be acceptable.

The residual pressure threshold may be chosen for example according to a maximum acceptable bolus which may occur after releasing the occlusion. Depending for example on the medical fluid used during an infusion, for example a medication or an enteral feeding solution, it may be known that a small bolus is acceptable as it does not cause any harm to a patient. Based on this acceptable bolus, then, the residual pressure threshold may be set. For example, if a volume of 0.1 ml is considered as an acceptable bolus after an occlusion release, the residual pressure threshold may be set such that at most the acceptable bolus is administered to the patient after releasing the occlusion.

The residual pressure threshold may be determined, in one aspect, using the following equation:

$$P_{residual}[bar] = \frac{V_{Bolus}[ml]}{C[ml/bar]}$$

Herein, $P_{residual}$ is the residual pressure threshold, $V_{Bolus}$ is the acceptable volume of a bolus after releasing the occlusion, and C is the compliance of the delivery line and/or the pumping mechanism.

The compliance is to be understood as a measure for the expansibility of the system, for example a cylindrical tube of a syringe used on the syringe pump and/or a delivery line extending between the pumping mechanism and the patient. Generally, the compliance indicates the change of volume per pressure and accordingly is stated for example in ml/bar. With respect to for example a tubing set, the compliance indicates by what volume a tube expands if the pressure increases by a certain margin.

The compliance for a system can be measured easily by subjecting a system, for example a tube set, to pressure and by measuring the change in volume.

The compliance may be a constant or may be defined by a (non-linear) relation depending on the pressure.

The compliance for a particular delivery line and the pumping mechanism may be stored in a storage device of the infusion device. The storage device may for example store a multiplicity of compliance values associated with a multiplicity of different syringes (in case of a syringe pump) and/or delivery lines. This is based on the finding that different syringes and different delivery lines generally have different compliances, depending on the structural built of the syringe and the delivery line and the materials used, for example. By storing different compliance values for different syringes and different delivery lines, when using a particular syringe and a particular delivery line the associated compliance value may be chosen and the residual pressure threshold may be computed accordingly.

If a particular type of syringe is not defined within the storage device, for example a default value may be used. If for example a syringe is used of a particular manufacturer having a predefined volume, for example 50 ml, a default value for a 50 ml syringe may be used if the particular syringe from the particular manufacturer is not known to the system.

By means of the above equation the residual pressure threshold is computed beforehand, i.e. prior to an actual infusion operation. For example, if 0.1 ml of fluid is considered as an acceptable bolus and if the line compliance is stored as being 1.75 ml/bar (which may be the total compliance, influenced for example by the syringe compliance, by the compliance of the extension line and by the mechanical elasticity of the syringe pump, which for example may have values of 1.2 ml/bar, 0.15 ml/bar and 0.4 ml/bar, respectively) then the residual pressure threshold is computed using the above equation to be 60 mbar.

Generally, during a forward pumping when using a syringe pump as an infusion device, for estimating the pressure from the measurement value taken by the sensor device (for example a sensor measuring the force applied to the piston of the syringe used on the syringe pump) the following equation is used:

$$P = \frac{F - F_0}{S}$$

Herein, P is the estimated pressure in the delivery line, F is the force value measured by the sensor device, F0 denotes a frictional force component of the syringe used on the syringe pump, and S denotes the effective surface by which the piston of the syringe acts onto fluid contained in the cylindrical tube of the syringe. Using this equation, the pressure in the delivery line can be derived during the regular infusion process, and the pressure is compared to the predefined pressure threshold to detect whether an occlusion is present or not.

During reverse pumping, in contrast, a different equation is beneficially used to estimate the pressure in the delivery line:

$$P = \frac{F + F_0}{S}$$

This takes into account that generally the friction force opposes the movement of the piston, causing the sign of the frictional force component F0 to change. Hence, for deriving the pressure in the delivery line during the reverse pumping a different equation is used as compared to the equation used for deriving the pressure during the regular forward pumping.

The second criteria corresponds to a volume target and takes into account that the volume that has been pumped in the forward direction after occurrence of the occlusion can estimated, hence allowing for an estimation of the volume that should be pumped in the reverse direction. As the second criteria it is observed whether a predetermined volume has been pumped in the reverse, upstream direction, the predetermined volume being computed using for example the following equation:

$$V[ml] = C[ml/bar] \cdot P_{thres}[bar] \cdot K$$

Herein, V is the predetermined volume, C is the compliance of the delivery line and/or the pumping mechanism, $P_{thres}$ is the pressure threshold used for the occlusion detection, and K is a constant factor.

This is based on the finding that generally the pressure in the delivery line is (almost) 0 during regular infusion in case no occlusion is present. If an occlusion occurs, the pressure starts building up until the pressure threshold is reached, the buildup in pressure being due to the occlusion. The pressure buildup (which can be assumed to be - in total - equal to the pressure threshold) is linked to the volume that has been pumped after occurrence of the occlusion by the compliance such that, when knowing the compliance, the volume can be computed.

This can then be used for the second criteria, for which it is observed whether the pumped value during the reverse pumping has reached the volume computed from the above equation. If this is the case, the second criteria it is fulfilled.

Herein, a constant factor K may be used in the above equation as a safety margin for computing the volume. For example, for the constant factor a value in the range between 0.5 and 2, for example 1.0 or 1.5, may be chosen.

In one aspect, the reverse pumping is stopped if exactly one of the at least two criteria is fulfilled. Hence, as soon as one criteria is fulfilled, the reverse pumping is stopped. Alternatively, it however is also conceivable that the reverse pumping is only stopped if all of the criteria are fulfilled. If more than two criteria are applied, it also is conceivable that the reverse pumping is stopped if a subset, for example two out of three criteria, are fulfilled.

In one aspect, in addition a third criteria may be observed. For example, as a third criteria it may be monitored whether a position of first detection of the occlusion has been reached, and if this is the case, the third criteria is assumed to be fulfilled. This third criteria in particular is applicable if the potential presence of an occlusion is detected at a very early stage, for example by observing an abnormal slope in the pressure. If in a method for the occlusion detection an abnormal slope in the pressure is detected and a flag is set at that position (of the piston of the syringe in case of a syringe pump or of the peristaltic pumping mechanism in case a volumetric pump) at which the abnormal slope has first occurred, it may be assumed that the reverse pumping should last until this position of first detection has been reached. The pumping mechanism hence is operated in the reverse direction until the estimated position at which the occlusion first occurred.

If more than two criteria are used, the reverse pumping may be stopped by combining the criteria suitably. For example, the reverse pumping may be stopped as soon as the first criteria or as soon as both the second and the third criteria is/are fulfilled.

The object is also achieved by an infusion device for administering a medical fluid to a patient, comprising:

- a pumping mechanism for pumping a fluid through a delivery line,
- a sensor device for measuring a measurement value indicative of a pressure in the delivery line,
- a processor device for controlling the pumping mechanism, the processor device being constituted to detect, from a rise of the pressure beyond a predefined pressure threshold during a pumping action of the pumping mechanism in a downstream direction, the presence of an occlusion in the delivery line, the processor device further being constituted to reverse, in case of an occlusion, the pumping mechanism to pump fluid in an upstream direction opposite the downstream direction for reducing the pressure in the delivery line.

Herein, the processor device is constituted to stop the reverse pumping of the fluid in the upstream direction if at least one of at least two criteria is fulfilled, wherein the processor device is constituted to monitor

- as a first criteria whether the pressure in the delivery line has fallen below a predetermined residual pressure threshold, and
- as a second criteria whether the volume pumped in the upstream direction during the reverse pumping action of the pumping mechanism has reached a predetermined volume computed based on the predefined pressure threshold used for the occlusion detection.

The advantages and advantageous embodiments described above for the method equally apply to the infusion device such that it shall be referred to the above.

It is to be noted that the infusion device may be for example a syringe pump or a volumetric pump. In a syringe pump, a piston is pushed by means of a pusher device into a cylindrical tube of the syringe in order to deliver a medical fluid from the cylindrical tube towards a delivery line connected to the cylindrical tube. In a volumetric pump a pumping mechanism acts in a peristaltic fashion on a pump segment of a delivery line, for example on a membrane of the pump segment, such that a fluid is pumped through the delivery line.

The idea of the invention shall subsequently be described in more detail with reference to the embodiments shown in the figures. Herein:

Fig. 1    shows a view of an infusion device constituted as a syringe pump;

Fig. 2    shows a schematic diagram of a pressure rise over time in case of an occlusion; and

Fig. 3    shows a view of an infusion device constituted as a volumetric (peristaltic) infusion pump.

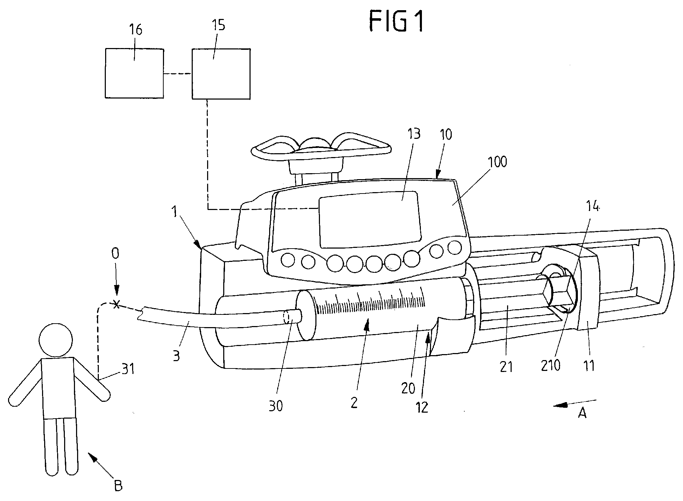

Fig. 1 shows an embodiment of an infusion device 1 in the shape of a syringe pump. The infusion device 1 comprises a housing 10 having a front face 100 and a display device 13 arranged thereon. The display device 13 may for example be a touch-sensitive display allowing a user to enter commands for operation of the infusion device 1 and displaying operational information regarding the process of an actual infusion operation.

The infusion device 1 comprises a receptacle 12 in which a syringe 2 having a cylindrical tube 20 is arranged. A piston 21 is movable within the cylindrical tube 20 and is in engagement with a pusher device 11 of a pumping mechanism of the infusion device 1. At an end of the cylindrical tube 20 opposite the piston 21 a delivery line 3 extends from the cylindrical tube 20 towards a patient B, the delivery line 3 being connected to the cylindrical tube 20 at an end 30 and to the patient B at an end 31.

The piston 21 comprises a head 210 facing away from the cylindrical tube 20 and being in abutment with the pusher device 11 of the infusion device 1. During operation of the infusion device 1, the pusher device 11 is electromotorically driven in an actuation direction A such that the piston 21 is moved into the cylindrical tube 20 and a medical fluid contained in the cylindrical tube 20 is delivered via the delivery line 3 towards the patient B.

The infusion device 1 comprises a processor device 15 and a storage device 16. Via the processor device 15 the infusion operation of the infusion device 1 is controlled. In the storage device 16 operational parameters, such as mechanical characteristics of the syringe 2 used on the infusion device 1 as well as operational data, may be stored.

During an infusion process a medical fluid, for example a medication or a nutritional fluid for the parenteral feeding of a patient or the like, is delivered from the cylindrical tube 20 via the delivery line 3 towards the patient B. For this, the piston 21 is continuously pushed into the cylindrical tube 20 in the actuation direction A such that a desired flow rate is obtained, which is programmed by a user prior to the start of the infusion operation.

The delivery line 3 generally is made of a flexible tubing made for example from a PVC material. The delivery line extends from the cylindrical tube 20 to the patient B and is, at its first end 30, in fluid connection with the cylindrical tube 20 and, at its second end 31, for example connected to a needle for providing an intravenous access to the patient B. During an infusion process an occlusion O in the delivery line 3 must be avoided and, if it nevertheless occurs, must be detected such that appropriate countermeasures to overcome the occlusion O can be taken. For this, a sensor device in the shape of a force sensor 14 is placed on the pusher device 11 facing the head 210 of the piston 214 measuring a force exerted on the piston 21 during an infusion process. From a force measured by means of the force sensor 14 an estimate of the pressure within the delivery line 3 connected to the syringe 2 can be obtained, such that the pressure within the delivery line 3 can be monitored. If it is found that the pressure within the delivery line 3 rises beyond a permissible threshold value, an alarm is triggered indicating that an occlusion O may be present in the system.

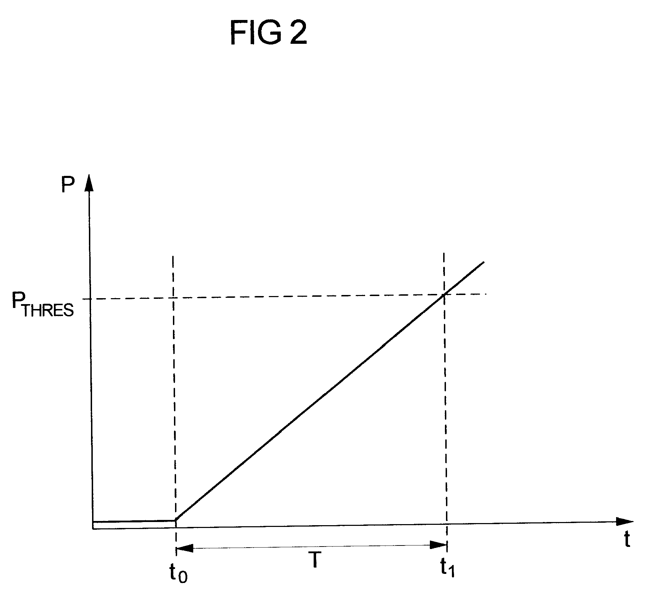

Fig. 2 shows in a schematic diagram the pressure P over time t in case of an occlusion O. Generally, the pressure P is very small (almost 0) during normal infusion operation in case no occlusion O is present (see the pressure P prior to the time t0). If at the time t0 an occlusion O occurs, the pressure P will start to rise and will continue to rise (if the occlusion O does not disappear) until a threshold value $P_{thres}$ is exceeded, at which moment an alarm is triggered by the processor device 15 such that a user is warned of the occlusion O.

The occlusion 0, in the example of Fig. 2, occurs at time t0. Until the pressure threshold value $P_{thres}$ actually is exceeded by the pressure curve P at time t1, a substantial time duration T may pass (corresponding to a distance by which the piston 21 is continued to be moved into the cylindrical tube 20), due to the continuous rise of the pressure P at a finite slope, the slope of the pressure rise herein depending on a multiplicity of factors, for example the flow rate and the compliance of the system, in particular the compliance of the cylindrical tube 20 of the syringe 2 and of the delivery line 3 extending in between the cylindrical tube 20 and the patient B.

As said, to observe the pressure in the delivery line 3, the force applied to the piston head 210 of the piston 21 by means of the pusher device 11 is measured by the sensor 14 placed in between the pusher device 11 and the piston head 210. The force measured in this way allows for an indirect measurement of the pressure within the cylindrical tube 20, which generally equals the pressure in the delivery line 3. In particular, the pressure in the cylindrical tube 20 depends on the measured force according to the following relation:

$$P = \frac{F - F_0}{S} .$$

Herein, P denotes the pressure, F denotes the measured force, $F_0$ denotes a frictional force component and S denotes the effective surface by which the piston 21 acts onto the fluid contained in the cylindrical tube 20. The effective surface S is substantially determined by the inner diameter of the cylindrical tube 20.

Whereas F is measured and S is known from the geometrical dimensions of the cylindrical tube 20 of the syringe 2, the frictional force component $F_0$ is subject to uncertainty. In particular, the frictional force component $F_0$ may vary in dependence on the specific syringe 2 used on the system, wherein the frictional force component $F_0$ generally is dependent on the position of the piston 21 within the cylindrical tube 20 and on the velocity by which the piston 21 is moved relative to the cylindrical tube 20 during an infusion process.

Hence, for a particular syringe of a particular model, a particular batch, a particular volume and a particular brand a specific frictional force may arise, possibly depending on the position of the piston 21 relative to the cylindrical tube 20 and on the velocity of movement. Generally, the frictional force may be obtained for example from a statistical analysis by measuring different syringes of different types, models and volumes with respect to their friction as a function of position of the piston 21 relative to the tube 20 and, possibly, also as a function of the velocity by which the piston 21 is moved relative to the tube 20. From such statistical analysis for example a mean frictional force associated with a particular syringe (of a particular type, model, brand and volume) may be stored in the storage device 16.

If it is found that the pressure in the delivery line 3 (derived by the above equation from the sensor readings of the force sensor 14) exceeds a predetermined threshold value, it is concluded that an occlusion is present, upon which an alarm is triggered and, potentially, the infusion is stopped. If now a user, for example a nurse, would suddenly release the occlusion, a bolus may occur due to the pressure buildup in the delivery line 3 because of the occlusion and may be administered to a patient, which generally shall be avoided.

Therefore, in case an occlusion is detected, the processor device 15 controls the pumping mechanism to drive to pusher device 11 in a reverse direction opposite to the actuation direction A such that a reverse pumping is initiated. This shall lead to a reduction of the pressure in the delivery line 3 such that an unwanted bolus is avoided.

The pressure reduction shall be such that the pressure in the delivery line 3 is reduced to an acceptable value, but at the same time a negative pressure in the delivery line 3 shall be avoided. Hence, the reverse pumping must be stopped when the pressure in the delivery line 3 is reduced sufficiently, but before a negative pressure can develop in the delivery line 3.

To stop the reverse pumping, it is proposed to take a multiplicity of criteria into account to ensure that the pressure is reduced sufficiently, but at the same time no negative pressure occurs in the delivery line 3. Herein, as a first criteria it is observed whether the pressure in the delivery line 3 has fallen below a predetermined residual pressure threshold. As a second criteria, it is determined whether the volume pumped in the upstream direction during the reverse pumping action of the pumping mechanism has reached a predetermined volume computed based on the predefined pressure threshold used for the occlusion detection. In addition, as a third criteria it may be taken into account whether a position of first detection of the occlusion has been reached.

The reverse pumping may be stopped as soon as one of the criteria is fulfilled, or the reverse pumping may be stopped by suitably combining the criteria. For example, the reverse pumping may be stopped if the first criteria is fulfilled or if the second and the third criteria are fulfilled.

The first criteria sets a pressure target. The first criteria is fulfilled as soon as the pressure in the delivery line 3 has fallen below a predetermined residual pressure threshold. This residual pressure threshold is chosen, in one embodiment, positive such that the first criteria is fulfilled before a negative pressure arises in the delivery line 3.

The residual pressure threshold, in one embodiment, is determined using the following equation:

$$P_{residual}\big[bar\big] = \frac{V_{Bolus}\big[ml\big]}{C\big[ml/bar\big]}$$

wherein $P_{residual}$ is the residual pressure threshold, $V_{Bolus}$ is the acceptable volume of a bolus after releasing the occlusion, and C is the compliance of the delivery line 3 and/or the pumping mechanism including the pusher device 11.

The computation of the residual pressure threshold hence takes into account the volume of the acceptable bolus, considering that the pressure in the delivery line 3 does not need to be reduced further than the residual pressure threshold corresponding to the acceptable bolus.

The compliance value C associated with the syringe 2 and the delivery line 3 is stored in the storage device 16 of the infusion device 1. The storage device 16 in this regard may store a multiplicity of compliance values C for different syringes 2 and different delivery lines 3 such that, by inputting for example the type of syringe 2 to the infusion device 1, the processor device 15 may refer to the compliance value C associated with the particular syringe 2 used on the system for computing the residual pressure threshold.

The storage device 16 may for example store a compliance value for a syringe 2 of a particular manufacturer and a particular volume. The storage device 16 may in addition store a default value for a syringe 2 of a particular volume, which may be used in case a particular syringe 2 of a particular manufacturer is not explicitly defined in the infusion device 1. A delivery line 3 may be identified by its length, its inner and/or outer diameter or the like and by its manufacturer, and associated with a particular type of delivery line 3 a particular compliance value may be stored in the storage device 16.

The storage device 16 may store a constant value for the compliance. Just as well it is conceivable that the storage device 6 stores a nonlinear relation for the compliance depending for example on the pressure in the system.

During the reverse pumping, hence, the pressure derived from the force sensor 14 is compared to the residual pressure threshold, and if the pressure falls below the residual pressure threshold, the first criteria is fulfilled. Herein, for deriving the pressure in the delivery line 3 from the sensor readings of the force sensor 14, a different equation than for the forward pumping is used, namely:

$$P = \frac{F + F_0}{S}$$

Hence, taking into account that the frictional force F0 opposes the movement and hence changes signs, for deriving the pressure P in the delivery line 3 during the reverse pumping the frictional force component F0 is used with opposite sign as compared to the equation noted above for the for the pumping in the forward, downstream pumping direction. Again, F is the force value measured by the force sensor 14, and S denotes the effective surface by which the piston 21 of the syringe 2 acts onto fluid contained in the cylindrical tube 20 of the syringe 2, determined by the inner parameter of the cylindrical tube 20.

The second criteria is a volume target, which may be determined using the following equation:

$$V[ml] = C[ml/bar] \cdot P_{thres}[bar] \cdot K$$

wherein V is the predetermined volume, C is the compliance of the delivery line 3 and/or the pumping mechanism including the pusher device 11, $P_{thres}$ is the pressure threshold used for the occlusion detection, and K is a constant factor.

This makes use of the assumption that the pressure buildup in the delivery line 3 during the occlusion approximately equals the pressure threshold, such that the volume that has been pumped in the downstream direction during the presence of the occlusion can be computed by multiplying the pressure threshold with the compliance of the system. Hence, during the reverse pumping approximately this volume should be pumped backwards in order to reduce the pressure in the delivery line 3, wherein the above noted equation takes a constant factor K into account, having a value of for example 1.5, in order to apply a safety margin.

The third criteria may be applied if the position at which (roughly) the occlusion first occurred is known. If for example an occlusion detection method is used which detects an abnormal slope in the pressure curve and sets a flag at the position of the first occurrence of the abnormal slope, during the reverse pumping it may simply be reverted back to the position of first detection, namely to the position of the flag at which first a abnormal slope in the pressure curve has been observed.

The different criteria may be combined in various ways. For example, the reverse pumping may be stopped as soon as one of the criteria is fulfilled. Or the reverse pumping may be stopped as soon as the first criteria or as soon as both the second and the third criteria are fulfilled.

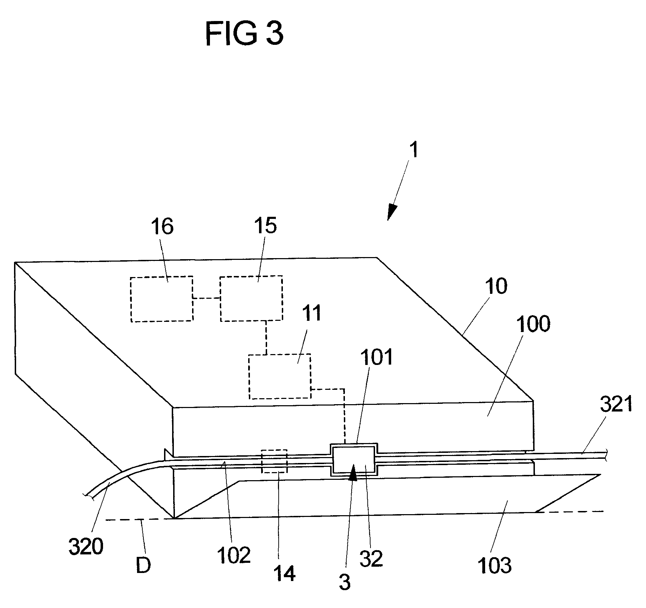

The invention is not limited to syringe pumps as shown in Fig. 1. Rather, as illustrated in Fig. 3, the invention likewise is applicable also to volumetric pumps.

In the example of Fig. 3, an infusion device 1 is constituted as a volumetric (peristaltic) infusion pump having a housing 10 and a front face 100 comprising a receptacle 101 in which a pump module 32 of an infusion set 3 can be received. A housing element 103 (also denoted as the "door") is pivotable about a pivoting axis D relative to the housing 10 and can be approached towards the front face 100 such that in a closed state the tubing set 3 is held on the infusion device 1.

From the pump module 32 tube sections 320, 321 extend. Of these tube sections 320, 321, an upstream tube section 321 connects the pump module 32 to a container containing a medical fluid, whereas a downstream pump section 320 connects the pump module 32 to a patient B for delivering the medical fluid towards the patient B. The infusion device 1 comprises a pump mechanism 11 (illustrated only schematically in Fig. 3) having for example a wobbling device acting onto the pump module 32 for peristaltically pumping a medical fluid through the tubing set 3 towards the patient B.

The invention may be implemented in the infusion device 1 according to Fig. 3 in an analogous fashion as described above, such that it shall be referred to the above. In the storage device 16 a compliance value of the tubing set 3 may be stored, wherein different compliance values for different tubing sets 3 of different types and of different manufacturers may be stored. The processor device 15 controls the operation of the infusion device 1 and, from the readings of a force sensor 14 measuring a force on the downstream tube section 320, the pressure P in the downstream tube section 320 may be determined and may be observed in order to detect an occlusion O in the system, and may also be observed to determine when to stop a reverse pumping to reduce the pressure in the tubing set 3 in case of an occlusion.

The idea of the instant invention is not limited to the embodiments described above, but may be carried out in an entirely different way in entirely different embodiments.

**List of Reference Numerals**

| | |
|---|---|
| 1 | Infusion device |
| 10 | Housing |
| 100 | Front face |
| 101 | Receptacle |
| 102 | Channel |
| 103 | Housing element (door) |
| 11 | Pusher device |
| 12 | Receptacle |
| 13 | Display device |
| 14 | Force sensor |
| 15 | Processor device |
| 16 | Storage device |
| 2 | Pumping device (syringe) |
| 20 | Cylindrical tube |
| 21 | Piston |
| 210 | Piston head |
| 3 | Delivery line |
| 30,31 | End |
| 32 | Pump module |
| 320, 321 | Tube section |
| A | Actuation direction |
| B | Patient |
| D | Pivoting axis |
| O | Occlusion |
| P | Pressure |
| $P_{thres}$ | Pressure threshold |
| t | time |
| T | Duration |

**Claims**

**1.** Method for operating an infusion device (1) for administering a medical fluid to a patient (P), comprising:

- pumping, using a pumping mechanism (11), a fluid in a downstream direction through a delivery line (3),
- measuring, using a sensor device (14), a measurement value indicative of a pressure in the delivery line (3) in order to be able detect, from a rise of the pressure beyond a predefined pressure threshold, the presence of an occlusion in the delivery line (3),
- in case of an occlusion, reversing the pumping mechanism (11) to pump fluid in an upstream direction opposite the downstream direction for reducing the pressure in the delivery line (3),

**characterized in**
**that** for stopping the reverse pumping of the fluid in the upstream direction at least two criteria are applied and the reverse pumping is stopped if at least one of the at least two criteria is fulfilled, wherein

- as a first criteria it is monitored whether the pressure in the delivery line (3) has fallen below a predetermined residual pressure threshold, and
- as a second criteria it is monitored whether the volume pumped in the upstream direction during the reverse pumping action of the pumping mechanism (11) has reached a predetermined volume computed based on the predefined pressure threshold used for the occlusion detection.

**2.** Method according to claim 1, **characterized in that** the residual pressure threshold is positive.

**3.** Method according to claim 1 or 2, **characterized in that** the residual pressure threshold is determined based on a maximum acceptable bolus which may occur after releasing the occlusion.

**4.** Method according to one of claims 1 to 3, **characterized in that** the residual pressure threshold is determined using the following equation:

$$P_{residual}[bar] = \frac{V_{Bolus}[ml]}{C[ml/bar]}$$

wherein $P_{residual}$ is the residual pressure threshold, $V_{Bolus}$ is the acceptable volume of a bolus after releasing the occlusion, and C is the compliance of the delivery line (3) and/or the pumping mechanism (11).

**5.** Method according to one of the preceding claims, **characterized in that**, if the infusion device (1) is a syringe pump, for estimating the pressure from the measurement value the following equation is used when pumping in the downstream direction:

$$P = \frac{F - F_0}{S}$$

and the following equation is used when pumping in the upstream direction:

$$P = \frac{F + F_0}{S}$$

wherein P is the estimated pressure in the delivery line (3), F is a force value measured by the sensor device (14), $F_0$ denotes a frictional force component of a syringe (2) used on the syringe pump, and S denotes the effective surface by which a piston (21) of the syringe (2) acts onto fluid contained in a cylindrical tube (20) of the syringe (2).

6. Method according to one of the preceding claims, **characterized in that** the predetermined volume is computed using the following equation:

$$V[ml] = C[ml/bar] \cdot P_{thres}[bar] \cdot K$$

wherein V is the predetermined volume, C is the compliance of the delivery line (3) and/or the pumping mechanism (11), $P_{thres}$ is the pressure threshold used for the occlusion detection, and K is a constant factor.

7. Method according to one of the preceding claims, **characterized in that** the reverse pumping is stopped as soon as one of the at least two criteria is fulfilled.

8. Method according to one of the preceding claims, **characterized in that** as a third criteria it is monitored whether a position of first detection of the occlusion has been reached during the reverse pumping.

9. Method according to claim 8, **characterized in that** the position of first detection is the position at which first an abnormal rise in pressure has been observed during the pumping in the downstream direction.

10. Method according to claim 8 or 9, **characterized in that** the reverse pumping is stopped when the first criteria or when the second and the third criteria is fulfilled.

11. Infusion device (1) for administering a medical fluid to a patient (P), comprising:

- a pumping mechanism (11) for pumping a fluid through a delivery line (3),
- a sensor device (14) for measuring a measurement value indicative of a pressure in the delivery line (3),
- a processor device (15) for controlling the pumping mechanism (11), the processor device (15) being constituted to detect, from a rise of the pressure beyond a predefined pressure threshold during a pumping action of the pumping mechanism (11) in a downstream direction, the presence of an occlusion in the delivery line (3), the processor device (15) further being constituted to reverse, in case of an occlusion, the pumping mechanism (11) to pump fluid in an upstream direction opposite the downstream direction for reducing the pressure in the delivery line (3),

**characterized in
that** the processor device (15) is constituted to stop the reverse pumping of the fluid in the upstream direction if at least one of at least two criteria is fulfilled, wherein the processor device (15) is constituted to monitor

- as a first criteria whether the pressure in the delivery line (3) has fallen below a predetermined residual pressure threshold, and
- as a second criteria whether the volume pumped in the upstream direction during the reverse pumping action of the pumping mechanism (11) has reached a predetermined volume computed based on the predefined pressure threshold used for the occlusion detection.

FIG 1

# FIG 2

# FIG 3

**EUROPEAN SEARCH REPORT**

Application Number

EP 16 30 5400

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 902 051 A1 (TERUMO CORP [JP]) 5 August 2015 (2015-08-05) * fig. 11; col. 20; whole document * | 1-11 | INV. A61M5/168 |
| X | US 2001/023345 A1 (WOLFF REMI [FR] ET AL) 20 September 2001 (2001-09-20) * par. 0097-0100; claims 20-22; whole document * | 1-11 | |
| X | EP 2 902 047 A1 (TERUMO CORP [JP]) 5 August 2015 (2015-08-05) * par. 0083, whole document * | 1-11 | |

TECHNICAL FIELDS
SEARCHED      (IPC)

A61M

The present search report has been drawn up for all claims

| Place of search | Date of completion of the search | Examiner |
|---|---|---|
| Munich | 15 September 2016 | Schindler-Bauer, P |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 16 30 5400

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2016

| Patent document cited in search report | | Publication date | Patent family member(s) | | Publication date |
|---|---|---|---|---|---|
| EP 2902051 | A1 | 05-08-2015 | EP | 2902051 A1 | 05-08-2015 |
| | | | JP | WO2014049647 A1 | 18-08-2016 |
| | | | WO | 2014049647 A1 | 03-04-2014 |
| US 2001023345 | A1 | 20-09-2001 | AT | 318156 T | 15-03-2006 |
| | | | DE | 60117310 T2 | 09-11-2006 |
| | | | EP | 1136089 A2 | 26-09-2001 |
| | | | ES | 2258040 T3 | 16-08-2006 |
| | | | FR | 2806310 A1 | 21-09-2001 |
| | | | US | 2001023345 A1 | 20-09-2001 |
| EP 2902047 | A1 | 05-08-2015 | CN | 104582752 A | 29-04-2015 |
| | | | EP | 2902047 A1 | 05-08-2015 |
| | | | JP | WO2014049661 A1 | 18-08-2016 |
| | | | US | 2015238689 A1 | 27-08-2015 |
| | | | WO | 2014049661 A1 | 03-04-2014 |

EPO FORM P0459

For more details about this annex : see Official Journal of the European Patent Office, No. 12/82